(19)

Europäisches
Patentamt
European
Patent Office
Office européen
des brevets

(11) **EP 2 743 852 A1**

(12) **EUROPEAN PATENT APPLICATION**
published in accordance with Art. 153(4) EPC

(43) Date of publication:
**18.06.2014 Bulletin 2014/25**

(21) Application number: **12820286.8**

(22) Date of filing: **31.07.2012**

(51) Int Cl.:
***G06F 19/24*** *(2011.01)*

(86) International application number:
**PCT/ES2012/070594**

(87) International publication number:
**WO 2013/017725 (07.02.2013 Gazette 2013/06)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.08.2011 ES 201131340**

(71) Applicants:
• **Universitat Autònoma De Barcelona**
 **08193 Bellaterra (Cerdanyola del Valles) (ES)**
• **Masaryk University**
 **601 77 Brno (CZ)**

(72) Inventors:
• **VALIENTE MALMAGRO, Manuel**
 **E-08193 Cerdanyola del Vallès (ES)**
• **LÓPEZ MESAS, Montserrat**
 **E-08193 Cerdanyola del Vallès (ES)**
• **ALONSO SÁNCHEZ, Agustín**
 **E-08193 Cerdanyola del Vallès (ES)**
• **HAVEL, Josef**
 **601 77 Brno (CZ)**

(54) **USE OF ARTIFICIAL NEURONAL NETWORKS FOR DETECTING THE FORMATION OF KIDNEY STONES AND FOR ASCERTAINING THE COMPOSITION OF SAID STONES**

(57) Use of artificial neural networks for detecting the formation of kidney stones and identifying the chemical composition of such stones, which comprises a multivariate analysis and artificial neural networks (ANN) on a database including from 10 to 14 metabolic markers in urine as analysis parameters selected from the group consisting of urine volume, pH, creatinine levels, uric acid, urea, sodium, potassium, chloride, citrate, calcium, oxalate, magnesium, phosphate and proteinuria.

EP 2 743 852 A1

**Description**

**FIELD OF THE INVENTION**

This invention, as stated above in the title, relates to the use of artificial neural networks for detecting the formation of kidney stones and identifying the chemical composition of such stones.

More particularly, the object of this invention is directed to a diagnostic method for the detection of renal lithiasis, which is based on the examination of different metabolic parameters in urine by means of artificial neural networks (ANN). This is an alternative method that improves classical parametric statistics and allows patterns to be recognized within complex data sets with almost absolute certainty, the invention advantageously helps elucidate whether a patient is or not a kidney stone former by means of the analysis of various metabolic markers in urine.

A further object of the invention is to use ANN and the above analysis of metabolic parameters in urine for establishing, if appropriate, the origin and composition of kidney stones and determining (96% accuracy) the type of stones formed. Because not all stones are similar, treatment for patients will depend on the type of kidney stones (calcium oxalate monohydrate, calcium oxalate dihydrate, hydroxyapatite, etc.).

Particularly, both for detection of kidney stones and identification of their chemical composition, the present invention provides artificial neural network analysis by using a certain and specific number of metabolic markers in urine which are as follows:

Urine volume, pH, creatinine levels, uric acid, urea, sodium, potassium, chloride, citrate, calcium, oxalate, magnesium, phosphate and proteinuria.

**BACKGROUND FOR THE INVENTION**

Urolithiasis is a common disease that is characterized by a high prevalence and incidence and a growing morbidity throughout the world. Up to 20% of population is affected by urolithiasis in some regions [1]. A five-year recurrence rate is 44-75% [2]. This fact is due to the persistence of a urinary disorder causing the formation of kidney stones. Such a disorder can be diagnosed and treated by dietary changes, pharmacological management or both, thus decreasing the disease severity and consequently the number of recurrences [3].

An accurate evaluation of the magnitude of kidney dysfunction is needed in order to apply an effective therapeutic treatment and diagnose the recurrence risk in each patient [4]. Also the risk of lithiasis in both healthy people and people who are more likely to develop this disease, i.e., people suffering from hypercalciuria, hyperoxaluria, hyperuricosuria, or renal tubular acidosis, should be evaluated and consequently preventive measures can be advantageously taken.

Considering the chemical composition of kidney stones, the most common components are calcium oxalate monohydrate or calcium oxalate dihydrate (63%), hydroxyapatite (7%), hydroxyapatite combined with oxalate calcium (11 %), uric acid (8%) and the remaining cases (11 %) from which struvite (4%) is the most representative. [5]

Nowadays the causes associated with each kidney stone are known. In previously published studies [6], the relative risk of kidney stone formation was reported to be high due to the increase or decrease in the levels of some urinary parameters, although no important relationship among all variables was established. For an urologist it is difficult to predict lithiasis episodes based on the examination of urine results, since different urine analytes are determined and multivariate data, which are usually analysed by individual parameters, are obtained.

In the 24-hour urine analysis, except in extreme cases, the diagnosis of any type of kidney stone, as discussed above, is not easy. On several occasions, a 24-hour urine collection has been reported not to be the most adequate for the evaluation of lithogen risk test [7] [8], since the changes in urinary parameters that could be used to differentiate stone formers (SF) and healthy controls (C) are masked. However, the 24-hour urine analysis is still used in most hospitals, thus underestimating other type of tests as for example the 8-hour urine analysis.

It is possible a better understanding of the system by analysing data collected from multivariate analysis techniques. In this aspect, artificial neural networks (ANN), which were developed in the 20th century and inspired from neurobiological basis of the human brain, can provide a classification after the systemic determination of unknown samples, since ANNs are able to recognize patterns from highly complex data sets [9]. At present, there is a large number of ANN applications in all fields of science, for example, chemistry, biochemistry, biology, as well as medicine. ANNs are specifically used in microbiology, ecology, hydrobiology, entomology, forensic clinic and extensively in medical diagnosis [10].

The objective of this invention is to examine and evaluate the discriminatory power of urolithiasis parameters by ANN in order to assess the possibility for classification of each parameter in urine, establish a methodology for prediction of suffering a lithiasis episode and contribute to a better knowledge of the disease physiopathology.

Regarding prior art, it should be noted that applicants have found some patents directed to lithiasis diagnosis. However, these documents relate to the detection of other parameters, for example, albumin in urine (EP 0504409B1) or vitamin D3 in plasma (RU2155347), or determination of the presence of polymorphism in osteopontin gene (JP2008000096), etc., which are based on metabolic parameters.

Moreover, there are different studies reporting the risk factors of lithiasis. Some of these studies describe the use of neural networks for determining the risks of developing kidney stones; among them the most important are as follows:

- Artificial neural networks for assessing the risk of urinary calcium stone among men. Dussol et al. Urol Res (2006) 34: 17-25. In this study, the ANNs are used to determine the risk of kidney stone formation, which is part of the present invention, but with the difference that determination is based in only some of the parameters used, i.e., age, body mass index (BMI), calcemia, calciuria, oxaluria, uricosuria, urea and sodium in urine. In addition, a very accurate diagnosis is not attained at all. Cut-off values are certainly determined.

- *Urinary analysis of nephrolithiasis markers. Barba et al.* Journal of Chromatography B, (2002) 433-455.. In this document, some of the biomarkers used in the present invention are described; these biomarkers are associated to kidney stone formation and some of them are useful to establish relationship with the type of stone formed.

- The role of urinary kidney stone inhibitors and promoters in the pathogenesis of calcium containing renal stones. Basavaraj et al., EAU-EBU Update series 5(2007) 126-136. This document provides a compilation of promoters and inhibitors of lithiasis including some of the markers proposed in the present invention.

- Nephrolithiasis. Bushinsky et al, JASN May 1, 1998 vol. 9 no. 5 917-924. This document comprises markers which are coincidental with those of the present invention. Particularly, urine volume, pH, calcium, phosphate, sodium, uric acid, oxalate, citrate, creatinine.

- Value of the urinary stone promoters/inhibitors ratios in the estimation of the risk of urolithiasis. Batinic et al., J Chem Inf Comput Sci 2000; 40:607-610. This document evaluates the levels of different markers by ANN in order to determine lithiasis risk.

Therefore, as the data analysis software is known and will not be an object of the present invention, and the use of ANN in some metabolic markers for determination of lithiasis risk is also known, the novelty of the present invention can be based on the following two scopes of protection: use of ANN with a certain group of markers for determining whether a patient is stone former and use of ANN with said markers for establishing the type of kidney stones. Therefore, while it is certain that not one of the proposed markers is new individually, it is also certain that the whole group of markers is new.

Moreover, regarding the first aspect of the present invention, although there are certain documents reporting the use of some of the markers of the present invention, the applicants provide solutions to the problem arisen that overcome any known drawback. This is due to the specific use of markers in the present invention that allow patients to be accurately diagnosed with a 100% success rate (this percentage has not yet been attained in Prior Art).

Regarding the second aspect of the present invention, no documents have been found reporting the description of groups of markers that can identify the composition/origin of renal stones. The present invention proposes the use of a specific and particular number of markers in order to determine the stone composition with a 96% success rate.

## DETAILED DESCRIPTION OF THE INVENTION

### Methodology

*Selection of patients with urinary lithiasis (stone formers; SF) and kidney stone analysis.*

Stone formers (SF) were recruited from patients attending the Lithotripsy Unit at Bellvitge University Hospital (BUH), Barcelona, Spain from January 2004 to January 2008, irrespectively of the number of lithiasis episodes suffered.

These stone formers had eliminated stones either spontaneously or by extracorporeal shock wave lithotripsy (ESWL). Their kidney stones were analysed by stereoscopic microscopy for the morphological and structural examination. This was complemented by scanning electron microscopy and energy-dispersing X-ray analysis (SEM-EDX) that allow microstructure observation and elemental analysis of microzones in the sample examined [11].

Following analysis, the stone formers were classified into four groups in accordance with the main stone component: calcium oxalate monohydrate (COM), calcium oxalate dihydrate (COD), hydroxyapatite (HAP) and uric acid (UA).

All patients were evaluated in order to exclude those suffering from systemic diseases, renal tubular acidosis, primary hyperoxaluria or urinary tract infections.

Metabolic assessment in stone formers was performed at least 2 months after their last lithiasis episode. Stone formers were not submitted to any dietary restriction or physical activity, and they had not been taking any medication that could affect their mineral metabolism.

Therefore, 133 stone formers were included in the study. Among these patients, 46 were classified as COM, 56 COD, 16 HAP and 16 UA.

*Control patients*

The control group, which was recruited from January 2004 and January 2008, consisted of 36 healthy volunteers who had no personal history of kidney stones and had not been taking any medication that could affect their mineral metabolism.

The control patients were examined for personal characteristics, and urine collections were accurately performed.

**Urine tests**

24-hour and 8-hour urine samples were collected in sterile containers containing thymol as a preservative and then refrigerated immediately. Urine volume was recorded after collection. A 2-hour urine collection was carried out after overnight fasting, and pH was immediately measured using a glass electrode (Crison pH-meter).

Sodium, potassium and chloride were determined by ion-selective electrode indirect potentiometry using the Modular Analytics (Roche Diagnostics) system.

Urea, creatinine, proteins, oxalate, citrate, calcium, phosphate, magnesium and uric acid were determined by molecular absorption spectrometry using the Modular Analytics (Roche Diagnostics) system.

Precision of each daily urine collection was evaluated on the basis of urine creatinine levels.

**Multivariate Analysis**

*ANN Calculation Program*

ANN calculation was performed using Trajan Neural Network Simulator, version 3.0 D. (Trajan Software Ltd 1996-1998, United Kingdom). All calculations were performed with a standard PC and Microsoft Windows XP Professional operative system.

ANN is a sophisticated tool of computational modelling that can be used to solve a wide variety of complex problems. The attraction of ANN comes from its learning capacity and/or modelling of very complex systems, as well as its possible use as a classification tool. Therefore, the real possibilities of ANN in science are high.

The difference between conventional computing programs and ANN is based on the fact that conventional software only performs the tasks for which it was specifically designed, while ANN is a form of machine learning system that enables, in principle, to learn almost everything. As the theory of ANN has been widely discussed in the literature, below is a brief description of the basic idea of this methodology for the only purpose of elucidating the necessary information.

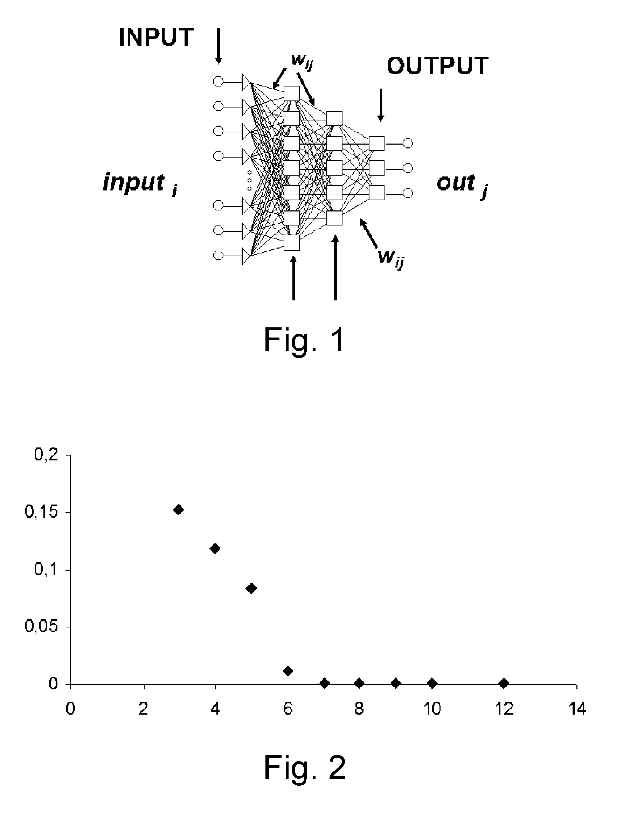

ANN is a computational model formed from a certain number of individual units, the artificial neurons or nodes, which are connected to weight coefficients $w_{ij}$ comprising the neural structure. Many neural network architectures can be used. Conventionally they are constituted by three or more layers, i.e., input, output or hidden layers (Fig. 1).

Each layer has a different number of nodes. The input layer receives information on the system (the nodes of this layer are simple distribution nodes that do not alter the input value at all). The information introduced in the input layer is modelled by the hidden layer, while the output layer is responsible for observation and behaviour. The $input_i$, multiplied by the connection weight $w_{ij}$, is first summed and then passed through a transfer function in order to produce the $output_i$. The determination of the appropriate number of hidden layers and the number of hidden nodes is one of the most important tasks in the design of ANN. In contrast to the input and output layers, there is no previous knowledge on the number and size of hidden layers.

The use of ANN consists of two steps: "training" and "prediction". In the first place, "training" involves the definition of input and output network data. Generally, it is necessary to adapt or standardize data to the network paradigm. These data are known as the training data set. In this training stage, where real data must be used, optimal structure, weight coefficients and network drawbacks are found out. Preparation is considered complete when the neural networks reach the required statistical precision, since they produce the necessary results for a specific input sequence. A good criterion to find the adequate network structure and therefore stop the learning process is to minimize the root mean square error (RMS) as shown is the following equation:

$$RMS = \sqrt{\frac{\Sigma_{i=1}^{N}\Sigma_{j-1}^{M}(y_{ij} - out_{ij})^2}{N \times M}}$$

where $y_{ij}$ is the matrix element (N x M) for the training or testing set, and $out_{ij}$ is the output matrix element (N x M) of neural network, where N is the number of variables in the model and M is the number of specimens. The RMS error provides a single number that abridges global error. After confirming that ANN works appropriately in training data, it is important to verify what ANN can do with data not seen before. This is called validation.

This test is fundamental to ascertain that the network not only has memorized the training set but also has learned the general patterns that participate within an application. At this stage, other input data are sent to the network in order to evaluate whether results can be predicted. In this case results are already known, but are not shown to the network. The expected value is compared with the experimental value to find that it is well functioning on the network. If the system does not give reasonably output data for this set of tests, the training period has not ended or the network is capable of modelling data but cannot be predicted. When validation of known data is the expected one, unknown data are evaluated and results are predicted in the final stage (classification of unknown data is made).

Data for calculation of ANN were divided at random: 1. Learning Set, 2. Validation Set, and 3. Testing Set.

The learning set consists of a series of samples (species) which is characterized by variables (characters) obtained by analysis of urine specimens. This set is used to design the adequate architecture of ANN from which classification is possible. The process in which the search of respective $w_{ij}$ weights is based with the aim of minimizing the RMS value is called learning or training. Accuracy of the model obtained with the most adequate data set, the architecture and training process, is also verified in another independent sample set of the so-called validation set. Finally, the ANN model obtained can be used to classify the mainly unknown species (testing set).

Each set represents a matrix with the number of columns that correspond to the number of variables. The variables not only can have numerical values (results from chemical analysis), but also nominal values (non-numerical), as well as output data. In the case of the present invention, output data were nominal, i.e., the name of the type of lithiasis (nomenclature).

It may happen that the matrix cannot be completed due to the fact that some variable values within data sets are not known or cannot be obtained. Although such cases that contain missing data are problematic, they can still be used in the analysis of data. There are several methods to manage missing data (e.g., using mean substitution, various types of interpolations and extrapolations).

## DESCRIPTION OF DRAWINGS

In order to complement the present invention and provide a better understanding of its distinguishing characteristics, the following figures are attached as an integral part of the description of this invention:

Figure 1 shows a general diagram of four-layer ANN architecture.

Figure 2 shows a cartesian diagram of the search for an optimal architecture of ANN for lithiasis detection, where the vertical axis indicates the root mean square error (RMS) and the horizontal axis indicates the number of nodes in hidden layers.

Figure 3 shows a diagram of an optimal architecture of ANN for lithiasis detection.

Figure 4 shows a cartesian diagram of the search for an optimal architecture of ANN for COD and COM classification.

Figure 5 shows a diagram of an optimal architecture of ANN for COD and COM classification.

## EXAMPLES

### - Case (1) 24-hour urine sample

*Preparation of database for the multivariate analysis with artificial neural networks (ANN).*

Input was represented by 14 parameters of urine analysis and as output in the classification of kidney stone formers and controls. Class 1 included the results from kidney stone formers and comprised 133 patients (COM, COD, HAP and UA formers), while Class 2 included the control group, namely, routine urine analysis from 36 healthy volunteers (without lithiasis).

*Search for ANN optimal architecture*

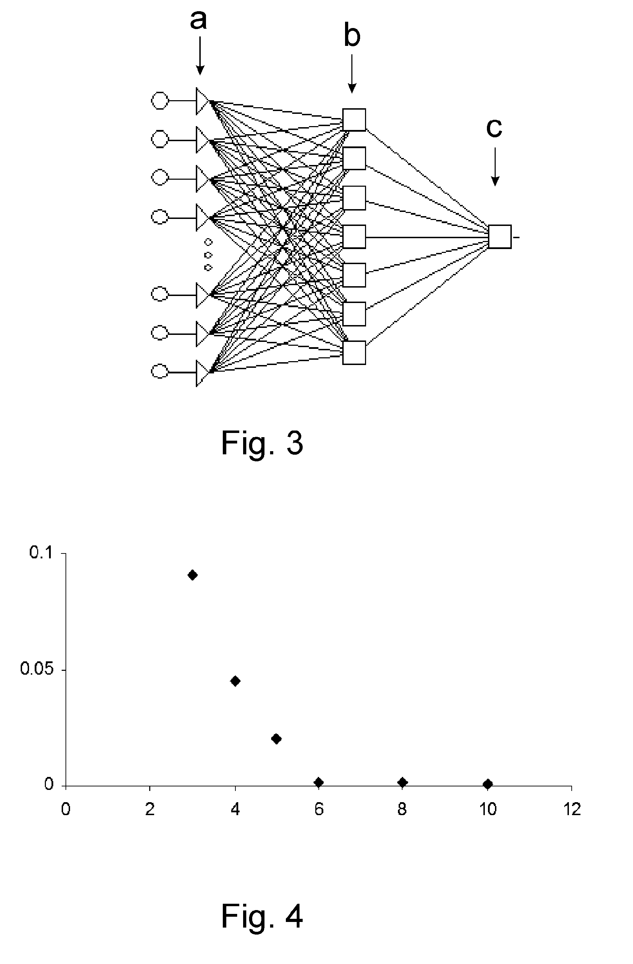

Whole data from the 24-hour urine analysis (169 cases) were submitted to ANN analysis. In the capacitation test, 26 cases were wrongly classified and, therefore, were removed. Thus, the total number of samples finally used in the ANN development was 115 and 28 (total 143). After removal, a search for an optimal architecture of ANN was performed again after the equation (1). It was found that the optimal number of nodes in the three-layer ANN structure was equal to 7 (Figure 2); therefore, the optimal architecture (Figure 3) was found to be 14, 7, 1, that is to say, number of inputs (quoted with (a)), number of nodes in the hidden layer (quoted with (b)), classification (quoted with (c)), and the classification attained in the capacitation test, for all urine samples, showed a 100% success rate.

*Cross-validation*

The cross-validation method was used to evaluate ANN and predictive error correction. Each sample was randomly removed from the training set, step by step, and its classification was verified. From 60 random cases comprising 48 samples of group 1, 12 samples from each COM, COD, HAP and UA group and 12 cases from group 0 attained a 96% success rate in classification.

Such an excellent result was at least surprising because the 24-hour urine has been reported to produce a masking effect, but the approach to ANN multivariate analysis, beyond any doubt, can potentially discriminate among cases and controls as well.

Another question is to distinguish the types of kidney stone formers by means of urine.

**- Case (2): 8-hour urine sample**

As noted above, calcium oxalate stone formers represent about 70% of patients with urolithiasis. By using a simple database and including only the controls and the COM/COD stone formers, there is a 72% success rate thus distinguishing between COM/COD stone formers and controls.

From the 8-hour night urine samples, which were collected by the subjects themselves in some cases only, 79 cases were similarly submitted to ANN analysis. The same 14 parameters in urine were represented in the input and output as the classification for COM/COD stone formers and controls.

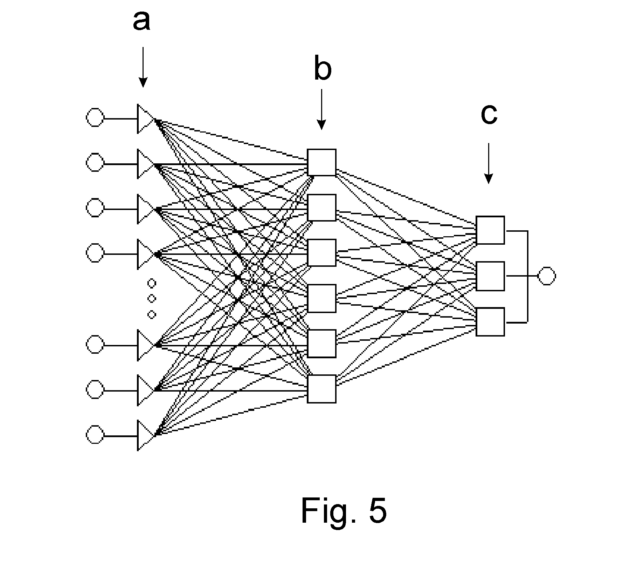

In the training test, it was necessary to remove six samples due to a wrong classification. Thus, the total number of samples used finally in the ANN development was 28 for COM stone formers, 30 for COD stone formers and 15 for controls (total 73). The optimal architecture of ANN was recorded again after the equation shown above in the section "Detailed Description of the Invention". It was found that the optimal number of nodes in this three-layer structure of ANN was equal to 6 (Figure 4). Elimination of "over-training" was assured and, therefore, prediction was reliable. Thus, the optimal architecture (Figure 5) was found to be 14, 6, 3, namely, number of inputs (a), number of nodes in the hidden layer (b), and classification (c); the classification acquired in the capacitation test showed a 100% success rate in all urine samples.

*Cross-validation*

After removal of outliers from the matrix, the cross-validation method was applied to verify ANN and prediction accuracy. In order to prove the classification and prediction power of ANN, all the samples, one by one, were randomly removed from the training set and used for verification of their classification. An 81 % success rate in classification was attained. Classification of COM and COD is possible and it should be emphasized that they represent almost 80% of cases.

Results were better in the cases from 8-hour urine collection -despite de smaller number of samples- than in the cases from 24-hour urine collection. The specific parameters or markers used for both urine collections were as follows:

Urine volume, pH, creatinine levels, uric acid, urea, sodium, potassium, chloride, citrate, magnesium, oxalate, calcium, phosphate and proteinuria.

*Conclusions*

The use of artificial neural networks for determination of the type of lithiasis was tested..

The variables selected in this study were found to be representative. These variables contain sufficient capacity for resolution and, therefore, sufficient capacity for the adequate classification and prediction of ANN, on the assumption that the number of samples in the training set be sufficiently large.

In general, as the number of samples (patients) is larger, prediction is better. Data in all the cases studied were successful, not only in ANN modelling but also in the prediction and identification of new and unknown species.

ANN development methods can be generally applied for any object to men from whom it is possible to determine adequately markers in a specific disease.

In conclusion, by means of a sufficiently extensive and reliable database, the use of artificial neural networks allows a rapid identification of the type of lithiasis and opens new possibilities for medical diagnosis.

Below is another comparative example showing significant differences in contrast to known systems because the complete set of markers is taken into account, which is a key factor for detection of urinary stone disease in multiparameters.

In this example, two different sets of parameters were used in 24-hour urine samples in order to evaluate robustness of the method claimed for diagnosis of kidney stones.

These two sets of variables were selected by taking into account:

Group 1 - The parameters reported for ephrolithiasis by Bushinsky et al, [JASN May 1, 1998 vol. 9 no. 5 917-924], where 9 urine parameters (urine volume, pH, creatinine, uric acid, sodium, citrate, oxalate, calcium and phosphate) were included for assessing the potential risk factors of urolithiasis.

Group 2 - In addition to the 9 urine parameters in the above group 1, 14 urine parameters from hospital protocol analysis (urine volume, pH, creatinine, uric acid, sodium, citrate, oxalate, calcium, phosphate, urea, proteinuria, potassium, chloride and magnesium) were also included.

*Diagnosis in stone-former patients or controlled patients (without healthy ex-patients).*

The total number of urine samples was 143, from which 115 were from stone formers and 28 from healthy subjects. Stone formers belonged to 4 major groups according to the main component of their stone, namely. 36 calcium oxalate monohydrate (COM), 47 calcium oxalate dihydrate (COD), 16 hydroxyapatite (HAP) and 16 Uric Acid (UA).

Input values of group 1 and group 2 were handled separately and submitted to artificial intelligence analysis. The output value represented the classification of stone former patients and healthy subjects. The classification attained in the preparation test for all urine samples resulted in a 100% success rate.

Fifty random cases comprising 40 samples from stone formers, 12 samples from each COM, COD, HAP and UA group, and 10 samples from controls were diagnosed for lithiasis. Diagnosis had only a success rate of 78% for group 1, while for group 2 the success rate was 96%, as shown in Table 1.

Table 1

| Diagnosis of lithiasis by ANN | | |
|---|---|---|
| | Group 1 | Group 2 |
| Variables o markers | Urine volume pH Creatinine Uric acid, Sodium Citrate Oxalate Calcium, Phosphate | Urine volume pH Creatinine Uric acid, Sodium Citrate Oxalate Calcium Phosphate Urea, Proteinuria Potassium, Chloride Magnesium |
| Success rate of diagnosis | 78% | 96% |

To sum up, the principal object of this invention is the use of artificial neural network (ANN) to detect kidney stones and determine their composition, which consists of a multivariate analysis and ANN using a database that includes, as analysis parameters, more than 9 and up to 14 metabolic markers in urine, namely:

> Urine volume
> pH
> Creatinine levels
> Uric acid
> Urea
> Sodium
> Potassium
> Chloride
> Citrate
> Calcium
> Oxalate
> Magnesium
> Phosphate
> Proteinuria.

Having sufficiently described the scope of the invention and the means to put it into practice, further disclosure is not necessary to enable a person skilled in the art to understand the scope and advantages of the invention. It will be apparent that the invention may be essentially practiced according to other embodiments differing in detail from consideration of the specification, whenever the main objective of the invention is not changed or modified.

## REFERENCES

[1]Daudon M.; Epidemiology of nephrolithiasis in France. Ann Urol 2005; 39:209-31.
[2] Ljunghall S, Danielson BG.; A prospective study of renal stone recurrences. Brit J Urol 1984; 56:122-124
[3] Coe FL, Parks JH, Asplin JR.; The pathogenesis and treatment of kidney stones. N Engl J Med 1992; 327:1141-1152.
[4] Tucak, Antun; Seric, Vatroslav; Kozmar, Dragutin; Sikiric, Maja; Zoric, Ivan; Babic-Ivancic, Vesna; Correlation of urine metabolic factors and urinary stones composition; Periodicum Biologorum (1999), 101(1), 35-44
[5] Grases, Felix; Costa-Bauza, Antonia; Ramis, Margarita; Montesinos, Vicente; Conte, Antonio. Simple classification of renal calculi closely related to their micromorphology and etiology. Clinica Chimica Acta (2002), 322(1-2), 29-36
[6] Bharathi, Pauline Suganthi Vijaya; Amirthaveni, M.; A comparative study of 24 hour urinary composition between urinary stone formers and healthy volunteers; Indian Journal of Nutrition and Dietetics (2007), 44(5), 250-260
[7] Parks Joan H; Goldfisher Evan; Asplin John R; Coe Fredric L; A single 24-hour urine collection is inadequate for the medical evaluation of nephrolithiasis; The Journal of urology (2002), 167(4), 1607-12.
[8] Seric, Vatroslav; Dutour-Sikiric, Maja; Mihaljevic, Ivan; Tucak-Zoric, Sandra; Bilic-Curcic, Ines; Babic-Ivancic, Vesna; Metabolic and physico-chemical urolithiasis parameters in the first morning urine ; Collegium Antropologicum (2009), 33(Suppl. 2), 85-92.
[9] Hernández-Borges et al. (2004) Applied ANN in chemotaxonomic classification of limpets (Patella)
[10] Volmer, Marcel; Wolthers, Bert G.; Metting, Harm J.; de Haan, Thijs H. Y.; Coenegracht, Pierre M. J.; van der Slik, Wim; Artificial neural network predictions of urinary calculus compositions analyzed with infrared spectroscopy; Clinical Chemistry (Washington, DC, United States) (1994), 40(9), 1692-7.
[11] Grases F, García-Ferragut L, Costa-Bauzá A. Analytical study of renal calculi. Recent Res Dev Pure. Appl Anal Chem 1998; 1:187-206

## Claims

1.    Use of artificial neural networks for detecting the formation of kidney stones and identifying the chemical composition of such stones, which comprises a multivariate analysis and artificial neural networks (ANN) on a database including at least 10 metabolic markers in urine as analysis parameters selected from the group consisting of urine volume, pH, creatinine levels, uric acid, urea, sodium, potassium, chloride, citrate, calcium, oxalate, magnesium, phosphate and proteinuria.

2. Use of artificial neural networks for detecting the formation of kidney stones and identifying the chemical composition of such stones, which comprises a multivariate analysis and artificial neural networks (ANN) on a database including from 10 to 14 metabolic markers in urine as analysis parameters selected from the group consisting of urine volume, pH, creatinine levels, uric acid, urea, sodium, potassium, chloride, citrate, calcium, oxalate, magnesium, phosphate and proteinuria.

3. Use of artificial neural networks for detecting the formation of kidney stones and identifying the chemical composition of such stones, which comprises a multivariate analysis and artificial neural networks (ANN) on a database including 14 metabolic markers in urine as analysis parameters selected from the group consisting of urine volume, pH, creatinine levels, uric acid, urea, sodium, potassium, chloride, citrate, calcium, oxalate, magnesium, phosphate and proteinuria.

INPUT

$w_{ij}$

OUTPUT

input $_i$

out $_j$

$w_{ij}$

## Fig. 1

0,2

0,15

0,1

0,05

0

0    2    4    6    8    10    12    14

## Fig. 2

Fig. 3

Fig. 4

Fig. 5

# INTERNATIONAL SEARCH REPORT

| International application No. |
| --- |
| PCT/ES2012/070594 |

## A. CLASSIFICATION OF SUBJECT MATTER

*G06F19/24* (2011.01)

According to International Patent Classification (IPC) or to both national classification and IPC

## B. FIELDS SEARCHED

Minimum documentation searched (classification system followed by classification symbols)
G06F

Documentation searched other than minimum documentation to the extent that such documents are included in the fields searched

Electronic data base consulted during the international search (name of data base and, where practicable, search terms used)

EPODOC, INVENES, WPI

## C. DOCUMENTS CONSIDERED TO BE RELEVANT

| Category* | Citation of document, with indication, where appropriate, of the relevant passages | Relevant to claim No. |
| --- | --- | --- |
| X | 1-2-2010, B.M.Singhal; "A Proposed Algorithm for Multivariate ArtificialNeural Network" International Journal of Computer Applications, Vol. 1, N° 3, Págs. 56-57 . Available in: http://www.ijcaonline.org/journal/number3/pxc387183.pdf | 1-3 |

☐ Further documents are listed in the continuation of Box C.　　☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance. | |
| "E" earlier document but published on or after the international filing date | |
| "L" document which may throw doubts on priority claim(s) or which is cited to establish the publication date of another citation or other special reason (as specified) | "X" document of particular relevance; the claimed invention cannot be considered novel or cannot be considered to involve an inventive step when the document is taken alone |
| "O" document referring to an oral disclosure use, exhibition, or other means. | "Y" document of particular relevance; the claimed invention cannot be considered to involve an inventive step when the document is combined with one or more other documents , such combination being obvious to a person skilled in the art |
| "P" document published prior to the international filing date but later than the priority date claimed | |
| | "&" document member of the same patent family |

| Date of the actual completion of the international search | Date of mailing of the international search report |
| --- | --- |
| 28/12/2012 | **(02/01/2013)** |
| Name and mailing address of the ISA/ OFICINA ESPAÑOLA DE PATENTES Y MARCAS Paseo de la Castellana, 75 - 28071 Madrid (España) Facsimile No.: 91 349 53 04 | Authorized officer M. Muñoz Sanchez Telephone No. 91 3495349 |

Form PCT/ISA/210 (second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0504409 B1
- RU 2155347
- JP 2008000096 B

### Non-patent literature cited in the description

- **DUSSOL et al.** Artificial neural networks for assessing the risk of urinary calcium stone among men. *Urol Res,* 2006, vol. 34, 17-25
- **BASAVARAJ et al.** The role of urinary kidney stone inhibitors and promoters in the pathogenesis of calcium containing renal stones. *EAU-EBU Update series,* 2007, vol. 5, 126-136
- **BUSHINSKY et al.** Nephrolithiasis. *JASN,* 01 May 1998, vol. 9 (5), 917-924
- **BATINIC et al.** Value of the urinary stone promoters/inhibitors ratios in the estimation of the risk of urolithiasis. *J Chem Inf Comput Sci,* 2000, vol. 40, 607-610
- **BUSHINSKY et al.** *JASN,* 01 May 1998, vol. 9 (5), 917-924
- **DAUDON M.** Epidemiology of nephrolithiasis in France. *Ann Urol,* 2005, vol. 39, 209-31
- **LJUNGHALL S ; DANIELSON BG.** A prospective study of renal stone recurrences. *Brit J Urol,* 1984, vol. 56, 122-124
- **COE FL ; PARKS JH ; ASPLIN JR.** The pathogenesis and treatment of kidney stones. *N Engl J Med,* 1992, vol. 327, 1141-1152
- **TUCAK, ANTUN ; SERIC, VATROSLAV ; KOZMAR, DRAGUTIN ; SIKIRIC, MAJA ; ZORIC, IVAN ; BABIC-IVANCIC, VESNA.** Correlation of urine metabolic factors and urinary stones composition. *Periodicum Biologorum,* 1999, vol. 101 (1), 35-44
- **GRASES, FELIX ; COSTA-BAUZA, ANTONIA ; RAMIS, MARGARITA ; MONTESINOS, VICENTE ; CONTE, ANTONIO.** Simple classification of renal calculi closely related to their micromorphology and etiology. *Clinica Chimica Acta,* 2002, vol. 322 (1-2), 29-36
- **BHARATHI ; PAULINE SUGANTHI VIJAYA ; AMIRTHAVENI, M.** A comparative study of 24 hour urinary composition between urinary stone formers and healthy volunteers. *Indian Journal of Nutrition and Dietetics,* 2007, vol. 44 (5), 250-260
- **PARKS JOAN H ; GOLDFISHER EVAN ; ASPLIN JOHN R ; COE FREDRIC L.** A single 24-hour urine collection is inadequate for the medical evaluation of nephrolithiasis. *The Journal of urology,* 2002, vol. 167 (4), 1607-12
- **SERIC, VATROSLAV ; DUTOUR-SIKIRIC, MAJA ; MIHALJEVIC, IVAN ; TUCAK-ZORIC, SANDRA ; BILIC-CURCIC, INES ; BABIC-IVANCIC, VESNA.** Metabolic and physico-chemical urolithiasis parameters in the first morning urine. *Collegium Antropologicum,* 2009, vol. 33 (2), 85-92
- **HERNÁNDEZ-BORGES et al.** *Applied ANN in chemotaxonomic classification of limpets (Patella,* 2004
- **VOLMER, MARCEL ; WOLTHERS, BERT G. ; METTING, HARM J. ; DE HAAN, THIJS H. Y. ; COENEGRACHT, PIERRE M. J. ; VAN DER SLIK, WIM.** Artificial neural network predictions of urinary calculus compositions analyzed with infrared spectroscopy. *Clinical Chemistry (Washington, DC, United States,* 1994, vol. 40 (9), 1692-7
- **GRASES F ; GARCÍA-FERRAGUT L ; COSTA-BAUZÁ A.** Analytical study of renal calculi. *Recent Res Dev Pure. Appl Anal Chem,* 1998, vol. 1, 187-206